# EUROPÄISCHE PATENTSCHRIFT

(11) **EP 1 775 276 B1**
(45) Veröffentlichungstag und Bekanntmachung des Hinweises auf die Patenterteilung: **29.08.2012**
(21) Anmeldenummer: 06019725.8
(22) Anmeldetag: 21.09.2006
(51) Int. Cl.: C07B 37/04, C07C 67/343

(54) **Verfahren zur Kupplung von Benzylaminen mit Halogenaromaten**
Process for the coupling of benzylamines and halogenated aromates
Procédé de couplage des benzylamines et aromates halogénés

(30) Priorität: 28.09.2005 DE 102005046344
(43) Veröffentlichungstag der Anmeldung: 18.04.2007
(73) Patentinhaber: Saltigo GmbH, 40764 Langenfeld (DE)
(72) Erfinder: Dockner, Michael, Dr., 50674 Köln (DE); Scholz, Ulrich, Dr., 45475 Mülheim an der Ruhr (DE); Neugebauer, Torsten, Dr., 53604 Bad Honnef (DE)
(74) Vertreter: Pettrich, Klaus-Günter

(56) Entgegenhaltungen:
- WO-A2-2004/052939
- WOLFE J P ET AL: "Simple, Efficient Catalyst System for the Palladium-Catalyzed Amination of Aryl Chlorides, Bromides, and Triflates" JOURNAL OF ORGANIC CHEMISTRY, AMERICAN CHEMICAL SOCIETY. EASTON, US, Bd. 65, Nr. 4, 2000, Seiten 1158-1174, XP002366818 ISSN: 0022-3263
- NORIYASU KATAOKA ET AL: "Air Stable, Sterically Hindered Ferrcenyl Dialkylphosphines for Palladium-Catalyzed C-C, C-N, and C-O Bond-Forming Cross-Coupling" JOURNAL OF ORGANIC CHEMISTRY, AMERICAN CHEMICAL SOCIETY. EASTON, US, Bd. 67, 4. Juli 2002 (2002-07-04), Seiten 5553-5566, XP002400233 ISSN: 0022-3263

## Beschreibung

Gegenstand der Erfindung ist ein Verfahren zur Kupplung von Benzylaminen mit Halogenaromaten zu den entsprechenden arylierten Benzylaminen unter Verwendung einer Base und eines Katalysators enthaltend eine Palladium-Verbindung und ein Bisaryl-dialkyiphosphin.

Arylierte Benzylamine finden vielfältige Verwendung als Synthesebausteine und Wirkstoffkomponenten, siehe. z.B. WO-A-02/00612.

Es ist bekannt, das sich arylierte Benzylamine durch Umsetzung von Anilinderivaten mit Benzylchloriden herstellen lassen (vgl. H.G.O. Becker, Organikum, 19. Aufl., Barth Dt. Verlag der Wiss. 1993, S.451, ISBN 3-335-00343-8). Nachteilig an diesem Verfahren ist es jedoch, dass entsprechend funktionalisierte Aniline bzw. Benzylchloride in vielen Fällen nur schwer zugänglich sind.

Die Herstellung arylierter Benzylamine mittels einer Palladium katalysierten Aminierungsreaktion von Chloraromaten ist beispielsweise in J. Org. Chem. 2002, 67, 5553-5566 oder von Bromaromaten in J. Org. Chem. 2000, 65, 1158-1174 beschrieben. Als Lösungsmittel kommen hierbei polare Lösungsmittel, wie z.B. Dimethoxyethan, oder unpolare, aprotische Lösungsmittel, wie z.B. Toluol, zur Verwendung. Durch eigene Untersuchungen wurde jedoch festgestellt, dass bei der Umsetzung mancher Edukte gemäß den beschriebenen Verfahren nur ein unvollständiger Umsatz zum gewünschten arylierten Benzylamin erreicht wird.

Es bestand demnach Bedarf, ein Verfahren zur Herstellung von arylierten Benzylaminen aus den entsprechenden Halogenaromaten und Benzylaminen zur Verfügung zu stellen.

Überraschenderweise wurde gefunden, dass die Herstellung arylierter Benzylamine aus den entsprechenden Halogenaromaten und Benzylaminen in guten Ausbeuten gelingt, wenn die Umsetzung in Gemischen aus unpolaren, aprotischen Lösungsmitteln und polaren, protischen oder aprotischen Lösungsmitteln erfolgt.

Gegenstand der vorliegenden Erfindung ist somit ein Verfahren zur Herstellung von N-(4-Cyclohex-1-enylphenyl)-aminomethylbenzocsäuremethylester
wobei 4-Aminomethylbenzoesäuremethylester
mit 4-Cyclohex-1-enylchlorbenzol umgesetzt werden, dadurch gekennzeichnet, dass die Umsetzung
- in Gegenwart von wenigstens einem Palladiumkomplex, der als Liganden wenigstens eine Verbindung der allgemeinen Formel (IV) trägt, 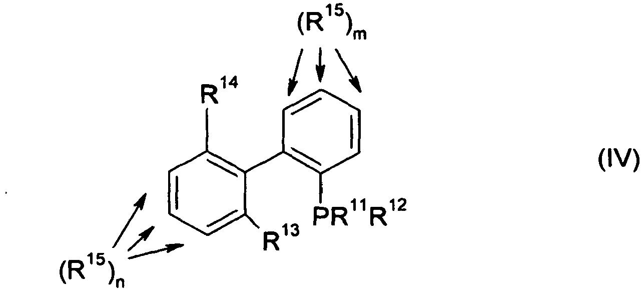 worin
   - R¹¹ und R¹²: jeweils unabhängig voneinander für C₁-C₁₂Alkyl oder C₅-C₁₉-Arylalkyl stehen,
   - R¹³ und R¹⁴: jeweils unabhängig voneinander für Wasserstoff, C₁-C₆-Alkyl, C₁-C₆-Dialkylamino oder C₁-C₆-Alkoxy stehen,
   - die Reste R¹⁵: jeweils unabhängig voneinander für Wasserstoff, C₁-C₁₂-Alkyl, C₁-C₁₂-Alkoxy, C₁-C₁₂-Fluoralkyl, C₁-C₁₂-Fluoralkoxy, Fluor, C₄-C₁₈-Aryl oder C₅-C₁₉-Arylalkyl stehen und die Pfeile die möglichen Bindungsstellen zum jeweiligen Arylrest anzeigen und
   - n und m: jeweils unabhängig voneinander für 0, 1, 2, oder 3 stehen und
   - •: in Gegenwart von Gemischen aus organischen Lösungsmitteln, enthaltend mindestens ein Lösungsmittel ausgewählt aus der Gruppe gegebenenfalls substituierter aromatischer Kohlenwasserstoffe und mindestens einem polaren Lösungsmittel ausgewählt aus der Gruppe Wasser, Amide, Ether, Alkohole oder tertiären Amine und
   - •: in Gegenwart von wenigstens einer alkalimetall- oder erdalkalimetallhaltigen Base erfolgt.

Unter **gegebenenfalls substituierten aromatischen Kohlenwasserstoffen** sind im Rahmen der Erfindung bevorzugt Chlor- oder C₁-C₆-Alkyl-substituierte aromatische Kohlenwasserstoffe zu verstehen. Als Lösungsmittel ausgewählt aus der Gruppe gegebenenfalls substituierter aromatischer Kohlenwasserstoffe eignen sich beispielsweise Benzol, Toluol oder Xylol.

Als **polare Lösungsmittel** eignen sich insbesondere Wasser, Amide, wie beispielsweise N,N-Dimethylformamid, N,N-Dimethylacetamid, N-Methyl-formanilid oder N-Methyl-pyrrolidon, Ether, wie beispielsweise Diethylether, Methyl-tert.-butylether, Diisopropylether, Dioxan, Tetrahydrofuran, Ethylenglykoldimethyl- oder -diethylether; Alkohole, wie beispielsweise Methanol, Ethanol, n- oder i-Propanol, tert.-Butanol, Ethylenglykolmonomethylether, Ethylenglykolmonoethylether, Diethylenglykolmonomethylether oder Diethylenglykolmonoethylether, oder tertiäre Amine, wie beispielsweise Tri-n-butylamin oder Triethylamin.

Bevorzugt sind Gemische, die mindestens ein polares Lösungsmittel ausgewählt aus Wasser, Amiden, Ethern, Alkoholen oder tertiären Aminen enthalten.

Besonders bevorzugt ist ein Gemisch enthaltend Toluol und wenigstens ein polares Lösungsmittel ausgewählt aus N,N-Dimethylformamid, N,N-Dimethylacetamid, N-Methyl-formanilid und N-Methyl-pyrrolidon.

Insbesondere bevorzugt sind Gemische enthaltend Toluol und N,N-Dimethylformamid.

Bevorzugt wird ein Gemisch aus mindestens 60 Vol.-% des gegebenenfalls substituierten aromatischen Kohlenwasserstoffes und höchstens 40 Vol.-% des polaren Lösungsmittels eingesetzt. In einer bevorzugten Ausführungsform des Verfahrens wird ein Gemisch aus mindestens zwei Volumenteilen des aromatischen Kohlenwasserstoffes und höchstens einem Volumenteil des polaren Lösungsmittels eingesetzt. Dabei kann es besonders bevorzugt sein, wenn das polare Lösungsmittel zudem mindestens zu 10 Vol.-% und der aromatische Kohlenwasserstoff zu höchsten 90 Vol.-% in dem Gemisch enthalten sind.

Insbesondere bevorzugt wird ein Gemisch aus 70 Vol.% Toluol und 30 Vol.% N,N-Dimethylformamid eingesetzt.

Der Umfang der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

**Alkyl** beziehungsweise **Alkoxy** steht jeweils unabhängig für einen geradkettigen, cyclischen, verzweigten oder unverzweigten Alkyl- beziehungsweise Alkoxy-Rest, wobei die genannten Reste gegebenenfalls weiter substituiert sein können. Gleiches gilt für den Alkylenteil eines Arylalkylrestes.

**C₁-C₆-Alkyl** steht beispielsweise und bevorzugt für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, **C₁-C₁₂-Alkyl** darüber hinaus z.B. für n-Heptyl, n-Octyl, n-Decyl und n-Dodecyl.

**C₁-C₆-Alkoxy** steht beispielsweise und bevorzugt für Methoxy, Ethoxy, n-Propoxy, iso-Propoxy, n-Butoxy, sec.-Butoxy, tert.-Butoxy, n-Pentoxy, cyclo-Hexoxy, cyclo-Pentoxy, n-Hexoxy, **C₁-C₁₂**-**Alkoxy** darüber hinaus z.B. für n-Heptoxy, n-Octoxy, n-Decoxy und n-Dodecoxy.

**Fluoralkyl** steht jeweils unabhängig für einen geradkettigen, cyclischen, verzweigten oder unverzweigten Alkyl-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

Aryl steht entweder für einen heteroaromatischen Rest mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sind oder für einen carbocyclischen aromatischen Rest mit 6 bis 18, bevorzugt 6 bis 10 Gerüstkohlenstoffatomen.

Beispiele für mono-, bi- oder tricyclische carbocyclische aromatische Reste mit 6 bis 18 Gerüstkohlenstoffatomen sind zum Beispiel Phenyl, Biphenyl, Naphtyl, Phenanthrenyl, Anthracenyl oder Fluorenyl, mono-, bi- oder tricyclische heteroaromatische Reste mit 5 bis 18 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuranyl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Fluor, Cyano, C₁-C₁₂-Alkyl, C₁-C₁₂-Fluoralkyl, C₁-C₁₂-Fluoralkoxy, C₁-C₁₂-Alkoxy oder Di(C₁-C₈-alkyl)amino.

**Arylalkyl** bedeutet jeweils unabhängig voneinander einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann. Ein Beispiel für Arylalkyl-Reste ist Benzyl.

Im Folgenden werden die bevorzugten Substitutionsmuster für die allgemeine Formel (IV) definiert:
- R¹¹ und R¹²: stehen bevorzugt jeweils unabhängig voneinander für iso-Propyl, tert-Butyl, Cyclopentyl, oder Cyclohexyl, besonders bevorzugt jeweils identisch für vorstehend genannte Reste und ganz besonders bevorzugt jeweils identisch für iso-Propyl,
- R¹³ und R¹⁴: stehen bevorzugt jeweils unabhängig für Methyl, Dimethylamino, Ethyl, iso-Propyl und Methoxy, besonders bevorzugt jeweils identisch für vorstehend genannte Reste und ganz besonders bevorzugt jeweils identisch für iso-Propyl,
- n: steht bevorzugt für 0 oder 1,
- m: steht bevorzugt für 0 und
- R¹⁵: steht bevorzugt jeweils unabhängig für Methyl, Ethyl, iso-Propyl oder Methoxy.

Eine besonders bevorzugte Verbindung der allgemeinen Formel (IV) ist 2-Dicyclohexylphosphino-2'-(N,N-dimethylamino)biphenyl.

Die Verbindungen der allgemeinen Formel (IV) sind literaturbekannt und beispielsweise gemäß S. Kaye, J. M. Fox, F. A. Hicks, S. L. Buchwald, Adv. Synth. Catal. 2001, 343, 8, 789-794. herstellbar.

Als Palladiumkomplexe, die als Liganden wenigstens eine Verbindung der allgemeinen Formel (IV) aufweisen, kommen beispielsweise isolierte oder präformierte Palladiumkomplexe enthaltend wenigstens eine Verbindung der allgemeinen Formel (IV) oder solche in Betracht, die durch Umsetzung eines Palladiumprecursors mit wenigstens einer Verbindung der allgemeinen Formel (IV) im Reaktionsmedium erzeugt werden. Vorzugsweise werden die für das Verfahren eingesetzten Palladiumkomplexe durch Umsetzung eines Palladiumprecursors mit wenigstens einer Verbindung der allgemeinen Formel (IV) im Reaktionsmedium erzeugt.

Geeignete Palladiumprecursoren sind alle Palladiumverbindungen, die mit Verbindungen der allgemeinen Formel (IV) unter Ausbildung einer Palladium-Phosphor-Koordination reagieren können.

Bevorzugte Palladiumprecursoren sind Pd₂(dibenzylidenaceton)₃, Allylpalladiumchlorid oder - bromid oder Palladiumverbindungen der allgemeinen Formel (Va),

PdY¹₂ (Va)

worin
- Y¹: für ein Anion, bevorzugt für Chlorid, Bromid, Acetat, Propionat, Nitrat, Methansulfonat, Trifluormethansulfonat, Acetylacetonat, Allyl oder Cyclopentadienyl steht,
oder Palladiumverbindungen der allgemeinen Formel (Vb),

PdY²₂L₂ (Vb)

worin
- Y²: für ein Anion, bevorzugt Chlorid, Bromid, Acetat, Methansulfonat oder Trifluormethansulfonat, Nonafluorbutansulfonat, Tetrafluoroborat oder Hexafluorophosphat steht,
- L: jeweils für ein Nitril, bevorzugt Acetonitril, Benzonitril oder Benzylnitril, oder ein Olefin, bevorzugt Cyclohexen oder Cycloocten, steht, oder
- L₂: zusammen für ein Diolefin, bevorzugt Norbomadien oder 1,5-Cyclooctadien steht.

Bevorzugt sind als Palladiumprecursoren Palladium(II)acetat oder [Pd₂(dba)₃].

Das molare Verhältnis von Palladium zu Verbindungen der allgemeinen Formel (IV) kann - insbesondere wenn die eingesetzten Palladiumkomplexe durch Umsetzung eines Palladiumprecursors mit wenigstens einer Verbindung der allgemeinen Formel (IV) im Reaktionsmedium erzeugt werden - beispielsweise 1 bis 4 betragen, bevorzugt jedoch 1,5 bis 3,5 und besonders bevorzugt 1,8 bis 3,2, insbesondere genau 2.

Das molare Verhältnis von Palladium zu 4-Cyclohex-1-enylchlorbenzol kann beispielsweise 0,000001 bis 0,05 betragen, bevorzugt jedoch 0,001 bis 0,03 und besonders bevorzugt 0,005 bis 0,02.

Das erfindungsgemäße Verfahren wird in Gegenwart von wenigstens einer alkalimetall- oder erdalkalimetallhaltigen Base durchgeführt. Alkalimetall- oder erdalkalimetallhaltige Basen sind beispielsweise und bevorzugt Alkalimetall- oder Erdalkalimetallhydroxide, -carbonate oder - alkanolate, besonders bevorzugt Alkalimetall- oder Erdalkalimetallhydroxide, wie beispielsweise Lithium-, Natrium-, Kalium- oder Calciumhydroxid, Alkalimetall- oder Erdalkalimetallcarbonate, wie beispielsweise Kalium- oder Caesiumcarbonat, oder Alkalimetallalkanolate, wie beispielsweise Natrium- oder Kalium-methanolat, -ethanolat, -n- oder -i-propanolat, -n-, -i-, -s- oder -t-butanolat. Ganz besonders bevorzugt sind Kaliumphosphat, Natrium- oder Kaliumhydroxid oder Natrium-oder Kalium-tertiär-butanolat. In einer bevorzugten Ausführungsform wird Kaliumphosphat verwendet.

Das molare Verhältnis von alkalimetall- oder erdalkalimetallhaltiger Base zu 4-Cyclohex-1-enylchlorbenzol kann beispielsweise 0,9 bis 1,5 betragen. Größere Mengen sind ebenfalls möglich, jedoch unwirtschaftlich. Bevorzugt beträgt das molare Verhältnis 1,0 bis 1,4 und besonders bevorzugt 1,2.

Das molare Verhältnis der Verbindung 4-Aminomethylbenzoesäuremethylester zu 4-Cyclohex-1-enylchlorbenzol kann beispielsweise 1 bis 2 betragen. Größere Mengen sind möglich, jedoch unwirtschaftlich. Bevorzugt beträgt das molare Verhältnis 1,0 bis 1,4 und besonders bevorzugt 1,1.

Die Reaktionstemperatur kann beispielsweise 40 bis 150°C, bevorzugt 60 bis 110°C, besonders bevorzugt 95 bis 105°C betragen, als Reaktionsdruck wird beispielsweise ein beliebiger Druck von 0,5 bis 100 bar gewählt; bevorzugt ist Umgebungsdruck.

Beliebige Reaktionszeiten sind geeignet. Beispielsweise kann die Reaktionszeit von 0,5 bis 48 h, bevorzugt 4 bis 24 h, besonders bevorzugt 6 bis 24 h betragen.

Auf die erfindungsgemäße Weise kann N-(4-Cyclohex-1-enylphenyl)-aminomethylbenzoesäuremethylester flexibler in höherer Reinheit und/oder höherer Ausbeute und/oder in einfacherer Weise erhalten werden als dies bisher mit den einleitend beschriebenen bekannten Verfahren möglich war.

Palladiumprecursors mit wenigstens einer Verbindung der allgemeinen Formel (IV) im Reaktionsmedium erzeugt werden - beispielsweise 1 bis 4 betragen, bevorzugt jedoch 1,5 bis 3,5 und besonders bevorzugt 1,8 bis 3,2, insbesondere genau 2.

Das molare Verhältnis von Palladium zu Verbindungen der allgemeinen Formel (III) kann beispielsweise 0,000001 bis 0,05 betragen, bevorzugt jedoch 0,001 bis 0,03 und besonders bevorzugt 0,005 bis 0,02.

Das erfindungsgemäße Verfahren wird in Gegenwart von wenigstens einer alkalimetall- oder erdalkalimetallhaltigen Base durchgeführt. Alkalimetall- oder erdalkalimetallhaltige Basen sind beispielsweise und bevorzugt Alkalimetall- oder Erdalkalimetallhydroxide, -carbonate oder - alkanolate, besonders bevorzugt Alkalimetall- oder Erdalkalimetallhydroxide, wie beispielsweise Lithium-, Natrium-, Kalium- oder Calciumhydroxid, Alkalimetall- oder Erdalkalimetallcarbonate, wie beispielsweise Kalium- oder Caesiumcarbonat, oder Alkalimetallalkanolate, wie beispielsweise Natrium- oder Kalium-methanolat, -ethanolat, -n- oder -i-propanolat, -n-, -i-, -s- oder -t-butanolat. Ganz besonders bevorzugt sind Kaliumphosphat, Natrium- oder Kaliumhydroxid oder Natrium- oder Kalium-tertiär-butanolat. In einer bevorzugten Ausführungsform wird Kaliumphosphat verwendet.

Das molare Verhältnis von alkalimetall- oder erdalkalimetallhaltiger Base zu Verbindungen der allgemeinen Formel (III) kann beispielsweise 0,9 bis 1,5 betragen. Größere Mengen sind ebenfalls möglich, jedoch unwirtschaftlich. Bevorzugt beträgt das molare Verhältnis 1,0 bis 1,4 und besonders bevorzugt 1,2.

Das molare Verhältnis der Verbindung der allgemeinen Formel (II) zu Verbindungen der allgemeinen Formel (III) kann beispielsweise 1 bis 2 betragen. Größere Mengen sind möglich, jedoch unwirtschaftlich. Bevorzugt beträgt das molare Verhältnis 1,0 bis 1,4 und besonders bevorzugt 1,1.

Die Reaktionstemperatur kann beispielsweise 40 bis 150°C, bevorzugt 60 bis 110°C, besonders bevorzugt 95 bis 105°C betragen, als Reaktionsdruck wird beispielsweise ein beliebiger Druck von 0,5 bis 100 bar gewählt; bevorzugt ist Umgebungsdruck.

Beliebige Reaktionszeiten sind geeignet. Beispielsweise kann die Reaktionszeit von 0,5 bis 48 h, bevorzugt 4 bis 24 h, besonders bevorzugt 6 bis 24 h betragen.

Auf die erfindungsgemäße Weise können Verbindungen der allgemeinen Formel (I) flexibler in höherer Reinheit und/oder höherer Ausbeute und/oder in einfacherer Weise erhalten werden als dies bisher mit den einleitend beschriebenen bekannten Verfahren möglich war.

### Beispiele

### Beispiel 1 (erfindungsgemäß):

In einem 1 1 Planschliffbecher mit Thermometer, Rückflußkühler und Septum werden 60 g 1-Chlor-4-cyclohex-1-enylbenzol, 72 g 4-(Aminomethyl)benzoesäuremethylester, 250 ml Toluol und 125 ml DMF vorgelegt. Unter Rühren werden 79,2 g Trikaliumphosphat eingetragen.

Die Apparatur wird jeweils dreimal evakuiert und wieder mit Stickstoff belüftet.

Anschließend gibt man bei Raumtemperatur eine Lösung von 0,7 g Palladiumacetat und 2,5 g 2-Dicyclohexylphosphino-2'-(N,N-dimethylamino)biphenyl gelöst in 7,0 g THF aus einem Rundkolben über eine Kanüle zu und erwärmt die Reaktionsmischung auf 110 °C.

Nach 22 Stunden bei 110 °C wird die Reaktionsmischung bei 80-90 °C filtriert. Der Filterkuchen wird zweimal mit jeweils 100 ml heißem Toluol (ca. 80 °C) gewaschen.

Die Filtrate werden vereinigt und bei Raumtemperatur zweimal mit jeweils 217 g einer 14 %igen Natriumchloridlösung gewaschen.

Die organische Phase wird im Vakuum aufkonzentriert. Zu dem Destillationssumpf werden 157 g Isopropanol gegeben. Durch Erwärmen auf 60 °C wird eine Lösung erhalten, die in 7-8 Stunden auf 20 °C abgekühlt wird. Das auskristallisierte Produkt wird abfiltriert. Der Filterkuchen wird zweimal mit jeweils 39 g kaltem Isopropanol gewaschen und schließlich im Vakuum bei 45 °C getrocknet.

Es werden 76 g (76 % der Theorie) Produkt isoliert.

### Beispiel 2 (nicht erfindungsgemäß):

In einem 1 1 Planschliffbecher mit Thermometer, Rückflußkühler und Septum werden 60 g 1-Chlor-4-cyclohex-1-enylbenzol, 72 g 4-(Aminomethyl)benzoesäuremethylester und 350 ml DMF vorgelegt. Unter Rühren werden 79,2 g Trikaliumphosphat eingetragen.

Die Apparatur wird jeweils dreimal evakuiert und wieder mit Stickstoff belüftet.

Anschließend gibt man bei Raumtemperatur eine Lösung von 0,7 g Palladiumacetat und 2,5 g 2-Dicyclohexylphosphino-2'-(N,N-dimethylamino)biphenyl gelöst in 7,0 g THF aus einem Rundkolben über eine Kanüle zu und erwärmt die Reaktionsmischung auf 110 °C.

Nach 22 Stunden bei 110 °C wird bei einer In-Prozess Kontrolle nur ein Umsatz von ca. 38% festgestellt, der sich auch bei längerer Rührzeit nicht weiter verändert. Die Reaktion wird abgebrochen.

### Beispiel 3 (nicht erfindungsgemäß):

In einem 1 1 Planschliffbecher mit Thermometer, Rückflußkühler und Septum werden 60 g 1-Chlor-4-cyclohex-1-enylbenzol, 72g 4-(Aminomethyl)benzoesäuremethylester und 350 ml Toluol vorgelegt. Unter Rühren werden 79,2 g Trikaliumphosphat eingetragen.

Die Apparatur wird jeweils dreimal evakuiert und wieder mit Stickstoff belüftet.

Anschließend gibt man bei Raumtemperatur eine Lösung von 0,7 g Palladiumacetat und 2,5 g 2-Dicyclohexylphosphino-2'-(N,N-dimethylamino)biphenyl gelöst in 7,0 g THF aus einem Rundkolben über eine Kanüle zu und erwärmt die Reaktionsmischung auf 110 °C.

Nach 22 Stunden bei 110 °C wird die Reaktionsmischung bei 80-90 °C filtriert. Der Filterkuchen wird zweimal mit jeweils 100ml heißem Toluol (ca. 80 °C) gewaschen.

Die Filtrate werden vereinigt und bei Raumtemperatur zweimal mit jeweils 217 g einer 14 %igen Natriumchloridlösung gewaschen.

Die organische Phase wird im Vakuum aufkonzentriert. Zu dem Destillationssumpf werden 157 g Isopropanol gegeben. Durch Erwärmen auf 60 °C wird eine Lösung erhalten, die in 7-8 Stunden auf 20 °C abgekühlt wird. Das auskristallisierte Produkt wird abfiltriert. Der Filterkuchen wird zweimal mit jeweils 39 g kaltem Isopropanol gewaschen und schließlich im Vakuum bei 45 °C getrocknet.

Es werden 41 g (41 % der Theorie) Produkt isoliert.

## Patentansprüche

1. Verfahren zur Herstellung von
N-(4-Cyclohex-1-enylphenyl)-aminomethylbenzoesäuremethylester wobei 4-Aminomethylbenzoesäuremethylester
mit 4-Cyclohex-1-enylchlorbenzol umgesetzt werden, **dadurch gekennzeichnet, dass** die Umsetzung
• in Gegenwart von wenigstens einem Palladiumkomplex, der als Liganden wenigstens eine Verbindung der allgemeinen Formel (IV) trägt, 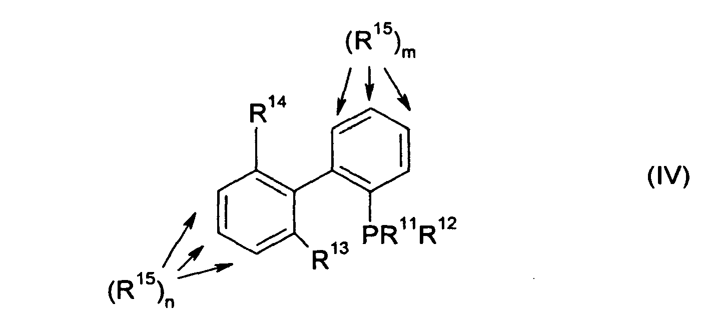
worin
R¹¹ und R¹² jeweils unabhängig voneinander für C₁-C₁₂-Alkyl oder C₅-C₁₉-Arylalkyl stehen,
R¹³ und R¹⁴ jeweils unabhängig voneinander für Wasserstoff, C₁-C₆-Alkyl, C₁-C₆-Dialkylamino oder C₁-C₆-Alkoxy stehen,
die Reste R¹⁵ jeweils unabhängig voneinander für Wasserstoff, C₁-C₁₂-Alkyl, C₁-C₁₂-Alkoxy, C₁-C₁₂-Fluoralkyl, C₁-C₁₂-Fluoralkoxy, Fluor, C₄-C₁₈-Aryl oder C₅-C₁₉-Arylalkyl stehen und die Pfeile die möglichen Bindungsstellen zum jeweiligen Arylrest anzeigen und
n und m jeweils unabhängig voneinander für 0, 1, 2, oder 3 stehen und
• in Gegenwart von Gemischen aus organischen Lösungsmitteln, enthaltend mindestens ein Lösungsmittel ausgewählt aus der Gruppe gegebenenfalls substituierter aromatischer Kohlenwasserstoffe und mindestens einem polaren Lösungsmittel ausgewählt aus der Gruppe Wasser, Amide, Ether, Alkohole oder tertiären Amine und
• in Gegenwart von wenigstens einer alkalimetall- oder erdalkalimetallhaltigen Base erfolgt.

2. Verfahren gemäß Anspruch 1, **dadurch gekennzeichnet, dass** das Gemisch aus organischen Lösungsmitteln mindestens einen aromatischen Kohlenwasserstoff ausgewählt aus Benzol, Toluol und Xylol, bevorzugt Toluol enthält.

3. Verfahren gemäß wenigstens einem der Ansprüche 1 bis 2, **dadurch gekennzeichnet, dass** das Gemisch aus 70 Vol.-% Toluol und 30 Vol.% N,N-Dimethylformamid besteht.

4. Verfahren gemäß wenigstens einem der Ansprüche 1 bis 3, **dadurch gekennzeichnet, dass** das Gemisch aus organischen Lösungsmitteln mindestens 60 Vol.% des aromatischen Kohlenwasserstoffes und höchstens 40 Vol.% des polaren Lösungsmittels enthält.

5. Verfahren gemäß wenigstens einem der Ansprüche 1 bis 4, **dadurch gekennzeichnet, dass** als Palladiumkomplexe, die als Liganden wenigstens eine Verbindung der allgemeinen Formel (IV) tragen, solche eingesetzt werden, die durch Umsetzung eines Palladiumprecursors mit wenigstens einer Verbindung der allgemeinen Formel (IV) im Reaktionsmedium erzeugt werden.

6. Verfahren gemäß wenigstens einem der Ansprüche 1 bis 5, **dadurch gekennzeichnet, dass** das molare Verhältnis von Palladium zu Verbindungen der allgemeinen Formel (IV) 1 bis 4 beträgt, bevorzugt 1,5 bis 3,5, besonders bevorzugt 1,8 bis 3,2 und insbesondere genau 2.

7. Verfahren gemäß wenigstens einem der Ansprüche 1 bis 6, **dadurch gekennzeichnet, dass** das molare Verhältnis von Palladium zu 4-Cyclohex-1-enylchlorbenzol 0,000001 bis 0,05 beträgt, bevorzugt 0,001 bis 0,03 und besonders bevorzugt 0,005 bis 0,02.

8. Verfahren gemäß wenigstens einem der Ansprüche 1 bis 7, **dadurch gekennzeichnet, dass** als alkalimetall- oder erdalkalimetallhaltige Basen Alkalimetall- oder Erdalkalimetallhydroxide, -carbonate oder -alkanolate eingesetzt werden.

9. Verfahren gemäß wenigstens einem der Ansprüche 1 bis 8, **dadurch gekennzeichnet, dass** das molare Verhältnis von alkalimetall- oder erdalkalimetallhaltiger Base zu 4-Cyclohex-1-enylchlorbenzol 0,9 bis 1,5 beträgt, bevorzugt 1,0 bis 1,4 und besonders bevorzugt 1,2.

10. Verfahren gemäß wenigstens einem der Ansprüche 1 bis 9, **dadurch gekennzeichnet, dass** das molare Verhältnis von 4-Aminomethylbenzoesäuremethylester zu 4-Cyclohex-1-enylchlorbenzol 1 bis 2 beträgt, bevorzugt 1,0 bis 1,4 und besonders bevorzugt 1,1.

## Claims

1. Process for preparing methyl N-(4-cyclohex-1-enylphenyl) aminomethylbenzoate by reacting methyl 4-aminomethylbenzoate with 4-cyclohex-1-enylchlorobenzene, **characterized in that** the reaction is effected
• in the presence of at least one palladium complex which bears, as ligands, at least one compound of the general formula (IV) 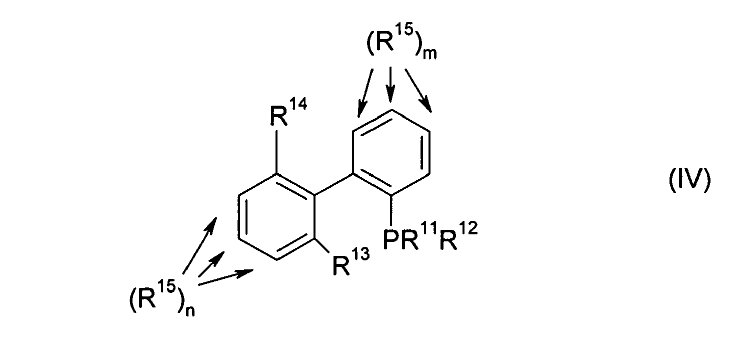 where
R¹¹ and R¹² are each independently C₁-C₁₂-alkyl or C₅-C₁₉-arylalkyl,
R¹³ and R¹⁴ are each independently hydrogen, C₁-C₆-alkyl, C₁-C₆-dialkylamino or C₁-C₆-alkoxy, the R¹⁵ radicals are each independently hydrogen, C₁-C₁₂-alkyl, C₁-C₁₂-alkoxy, C₁-C₁₂-fluoroalkyl, C₁-C₁₂-fluoroalkoxy, fluorine, C₄-C₁₈-aryl or C₅-C₁₉-arylalkyl, and the arrows indicate the possible bonding sites to the particular aryl radical, and
n and m are each independently 0, 1, 2, or 3
and
• in the presence of mixtures of organic solvents comprising at least one solvent selected from the group consisting of optionally substituted aromatic hydrocarbons and at least one polar solvent selected from the group consisting of water, amides, ethers, alcohols and tertiary amines
and
• in the presence of at least one alkali metal- or alkaline earth metal-containing base.

2. Process according to Claim 1, **characterized in that** the mixture of organic solvents comprises at least one aromatic hydrocarbon selected from benzene, toluene and xylene, preferably toluene.

3. Process according to at least one of Claims 1 to 2, **characterized in that** the mixture consists of 70% by volume of toluene and 30% by volume of N,N-dimethylformamide.

4. Process according to at least one of Claims 1 to 3, **characterized in that** the mixture of organic solvents contains at least 60% by volume of the aromatic hydrocarbon and at most 40% by volume of the polar solvent.

5. Process according to at least one of Claims 1 to 4, **characterized in that** the palladium complexes used which bear, as ligands, at least one compound of the general formula (IV) are those which are obtained by reacting a palladium precursor with at least one compound of the general formula (IV) in the reaction medium.

6. Process according to at least one of Claims 1 to 5, **characterized in that** the molar ratio of palladium to compounds of the general formula (IV) is 1 to 4, preferably 1.5 to 3.5, more preferably 1.8 to 3.2 and in particular exactly 2.

7. Process according to at least one of Claims 1 to 6, **characterized in that** the molar ratio of palladium to 4-cyclohex-1-enylchlorobenzene is 0.000001 to 0.05, preferably 0.001 to 0.03 and more preferably 0.005 to 0.02.

8. Process according to at least one of Claims 1 to 7, **characterized in that** the alkali metal- or alkaline earth metal-containing bases used are alkali metal or alkaline earth metal hydroxides, alkali metal or alkaline earth metal carbonates, or alkali metal or alkaline earth metal alkoxides.

9. Process according to at least one of Claims 1 to 8, **characterized in that** the molar ratio of alkali metal- or alkaline earth metal-containing base to 4-cyclohex-1-enylchlorobenzene is 0.9 to 1.5, preferably 1.0 to 1.4 and more preferably 1.2.

10. Process according to at least one of Claims 1 to 9, **characterized in that** the molar ratio of methyl 4-aminomethylbenzoate to 4-cyclohex-1-enylchlorobenzene is 1 to 2, preferably 1.0 to 1.4 and more preferably 1.1.

## Revendications

1. Procédé pour la préparation de N-(4-cyclohex-1-énylphényl)-aminométhylbenzoate de méthyle dans lequel on fait réagir du 4-aminométhylbenzoate de méthyle avec du 4-cyclohex-1-énylchlorobenzène, **caractérisé en ce que** la réaction s'effectue
• en présence d'au moins un complexe de palladium qui porte en tant que ligands au moins un composé de formule générale (IV), 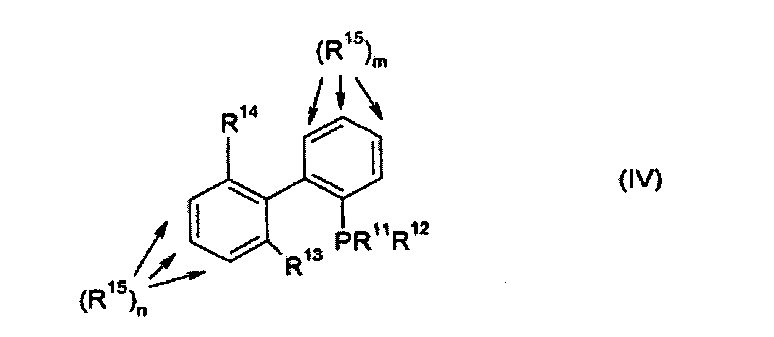 dans laquelle
R¹¹ et R¹² représentent chacun, indépendamment l'un de l'autre, un groupe alkyle en C₁-C₁₂ ou arylalkyle en C₅-C₁₉,
R¹³ et R¹⁴ représentent chacun, indépendamment l'un de l'autre, un atome d'hydrogène, un groupe alkyle en C₁-C₆, dialkylamino en C₁-C₆ ou alcoxy en C₁-C₆, les radicaux R¹⁵ représentent chacun, indépendamment les uns des autres, un atome d'hydrogène ou de fluor, un groupe alkyle en C₁-C₁₂, alcoxy en C₁-C₁₂, fluoroalkyle en C₁-C₁₂, fluoroalcoxy en C₁-C₁₂, aryle en C₄-C₁₈ ou arylalkyle en C₅-C₁₉ et les flèches indiquent les points de fixation au radical aryle respectif et
n et m représentent chacun, indépendamment l'un de l'autre, 0, 1, 2 ou 3
et
• en présence de mélanges de solvants organiques, contenant au moins un solvant choisi dans le groupe des hydrocarbures aromatiques éventuellement substitués et au moins un solvant polaire choisi dans le groupe constitué par l'eau, des amides, des éthers, des alcools ou des amines tertiaires
et
• en présence d'au moins une base contenant un métal alcalin ou un métal alcalino-terreux.

2. Procédé selon la revendication 1, **caractérisé en ce que** le mélange de solvants organiques contient au moins un hydrocarbure aromatique choisi parmi le benzène, le toluène et le xylène, de préférence le toluène.

3. Procédé selon au moins l'une des revendications 1 et 2, **caractérisé en ce que** le mélange consiste en 70 % en volume de toluène et 30 % en volume de N,N-diméthylformamide.

4. Procédé selon au moins l'une des revendications 1 à 3, **caractérisé en ce que** le mélange de solvants organiques contient au moins 60 % en volume de l'hydrocarbure aromatique et au maximum 40 % en volume du solvant polaire.

5. Procédé selon au moins l'une des revendications 1 à 4, **caractérisé en ce qu'**on utilise comme complexes de palladium, qui portent comme ligands au moins un composé de formule générale (IV), ceux qui sont produits dans le milieu réactionnel par la réaction d'un précurseur de palladium avec au moins un composé de formule générale (IV).

6. Procédé selon au moins l'une des revendications 1 à 5, **caractérisé en ce que** le rapport molaire du palladium aux composés de formule générale (IV) vaut de 1 à 4, de préférence de 1,5 à 3,5, de façon particulièrement préférée de 1,8 à 3,2 et en particulier exactement 2.

7. Procédé selon au moins l'une des revendications 1 à 6, **caractérisé en ce que** le rapport molaire du palladium au 4-cyclohex-1-énylchlorobenzène vaut de 0,000001 à 0,05, de préférence de 0,001 à 0,03 et de façon particulièrement préférée de 0,005 à 0,02.

8. Procédé selon au moins l'une des revendications 1 à 7, **caractérisé en ce qu'**on utilise comme bases contenant des métaux alcalins ou des métaux alcalino-terreux des hydroxydes, carbonates ou alcanolates de métaux alcalins ou de métaux alcalino-terreux.

9. Procédé selon au moins l'une des revendications 1 à 8, **caractérisé en ce que** le rapport molaire de la base contenant un métal alcalin ou un métal alcalino-terreux au 4-cyclohex-1-énylchlorobenzène vaut de 0,9 à 1,5, de préférence de 1,0 à 1,4 et de façon particulièrement préférée 1,2.

10. Procédé selon au moins l'une des revendications 1 à 9, **caractérisé en ce que** le rapport molaire du 4-aminométhylbenzoate de méthyle au 4-cyclohex-1-énylchlorobenzène vaut de 1 à 2, de préférence de 1,0 à 1,4 et de façon particulièrement préférée 1,1.
